(19) Europäisches Patentamt
European Patent Office
Office européen des brevets

(11) EP 0 790 244 A1

(12) **EUROPÄISCHE PATENTANMELDUNG**

(43) Veröffentlichungstag:
**20.08.1997 Patentblatt 1997/34**

(21) Anmeldenummer: **97101830.4**

(22) Anmeldetag: **06.02.1997**

(51) Int. Cl.$^{6}$: **C07D 295/14**, C07C 255/03, C11D 1/58, C11D 1/62, C11D 3/39

(84) Benannte Vertragsstaaten:
**AT BE CH DE DK ES FR GB IT LI NL PT SE**

(30) Priorität: **15.02.1996 DE 19605526**

(71) Anmelder: **HOECHST AKTIENGESELLSCHAFT
65929 Frankfurt am Main (DE)**

(72) Erfinder: **Löffler, Matthias, Dr.
65527 Niedernhausen (DE)**

# (54) Ammoniumnitrile und deren Verwendung als Bleichaktivatoren

(57) Ammoniumnitrile der allgemeinen Formel

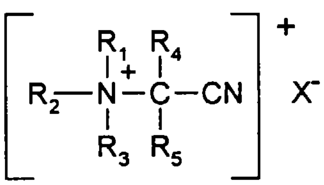

worin die Reste $R_1$ und $R_2$ zusammen mit dem Stickstoffatom einen Heterocyclus bilden. $R_3$ ist ein organischer Substituent, $R_4$ und $R_5$ sind entweder Wasserstoff oder weitere organische Substituenten, $R_3$ ist vorzugsweise ein niedriger Alkylrest, $R_4$ und $R_5$ sind vorzugsweise Wasserstoff, und X ist ein geeignetes Anion. Die erfindungsgemäßen Verbindungen werden als Bleichaktivatoren in bleichenden Wasch- und Reinigungsmitteln eingesetzt.

EP 0 790 244 A1

Printed by Rank Xerox (UK) Business Services
2.14.12/3.4

## Beschreibung

Diese Erfindung betrifft Ammoniumnitrile und Waschmittelzusammensetzungen, die diese Nitrile als Bleichaktivatoren enthalten.

Es ist bekannt, daß das Bleichvermögen peroxidischer Bleichmittel, wie Perborate, Percarbonate, Persilicate und Perphosphate, verbessert werden kann, so daß die Bleichwirkung bei niedrigeren Temperaturen einsetzt, etwa bei oder unter 60°C, indem man die Vorstufen von bleichenden Peroxysäuren zusetzt, die oft als Bleichaktivatoren bezeichnet werden.

Viele Substanzen sind nach dem Stand der Technik als Bleichaktivatoren bekannt. Gewöhnlich handelt es sich dabei um reaktive organische Verbindungen mit einer O-Acyl- oder N-Acyl-Gruppe, die in alkalischer Lösung zusammen mit einer Quelle für Wasserstoffperoxid die entsprechenden Peroxysäuren bilden.

Repräsentative Beispiele für Bleichaktivatoren sind etwa N,N,N',N'-Tetraacetylethylendiamin (TAED), Glucosepentaacetat (GPA), Xylosetetraacetat (TAX), Natrium-4-benzoyloxybenzolsulfonat (SBOBS), Natriumtrimethylhexanoyloxybenzolsulfonat (STHOBS), Tetraacetylglycoluril (TAGU), Tetraacetylcyansäure (TACA), Di-N-acetyldimethylglyoxin (ADMG) und 1-Phenyl-3-acetylhydantoin (PAH). Es sei beispielsweise auf GB-A-836 988, GB-A-907 356, EP-A-0 098 129 und EP-A-0 120 591 verwiesen.

Mittlerweise haben kationische Bleichaktivatoren, die eine quartäre Ammoniumgruppe enthalten, an Bedeutung gewonnen, da sie hocheffektive Bleichaktivatoren sind. Solche kationischen Bleichaktivatoren sind beispielsweise in GB-A-1 382 594, US-A-4 751 015, EP-A-0 284 292 und EP-A-0 331 229 beschrieben.

Ammoniumnitrile der Formel

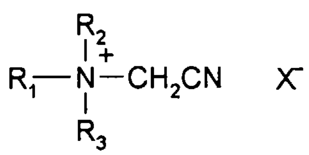

bilden dabei eine besondere Klasse kationischer Bleichaktivatoren. Verbindungen dieser Art und deren Verwendung als Bleichaktivator in Bleichmitteln sind beschrieben in EP-A-303 520, EP-A-464 880, EP-A-458 396 und US-A-4 883 917. Bei allen dort beschriebenen Verbindungen ist das Stickstoffatom der Ammoniumgruppe ausschließlich durch Alkyl-, Alkenyl- oder Arylgruppen substituiert.

Wahrscheinlich bilden diese Verbindungen bei der Perhydrolyse eine Peroxyimidsäure, welche das bleichende Agens ist.

Überraschenderweise wurde nun gefunden, daß Ammoniumnitrile der zuvor beschriebenen Art, die von cyclischen Aminen abgeleitet sind, eine bessere Bleichwirkung aufweisen als Nitrile gemäß dem Stand der Technik.

Gegenstand der Erfindung sind somit Verbindungen der allgemeinen Formel

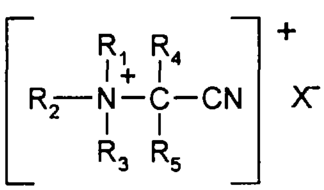

worin $R_1$ und $R_2$ zusammen mit dem Stickstoffatom, an das sie gebunden sind, einen Ring mit 4 bis 6 Kohlenstoffatomen bilden, der durch $C_1$- bis $C_5$-Alkyl, $C_1$- bis $C_5$-Alkoxy, $C_1$- bis $C_5$-Alkanoyl, Phenyl, Amino, Ammonium, Cyano, Cyanamino, Chlor oder Brom substituiert sein kann und wobei dieser Ring zusätzlich zum Stickstoffatom anstelle von Kohlenstoffatomen ein oder zwei Sauerstoff- oder Stickstoffatome, eine Gruppe N-$R_6$ oder eine Gruppe $R_3$-N-$R_6$ enthalten kann, worin $R_6$ Wasserstoff, $C_1$- bis $C_5$-Alkyl, $C_2$- bis $C_5$-Alkenyl, $C_2$- bis $C_5$-Alkinyl, Phenyl, $C_7$- bis $C_9$-Aralkyl, $C_5$- bis $C_7$-Cycloalkyl, $C_1$- bis $C_5$-Alkanoyl, Cyanomethyl oder Cyan ist,

$R_3$ ist $C_1$- bis $C_{24}$-, vorzugsweise $C_1$-$C_4$-Alkyl, $C_2$-$C_{24}$-, vorzugsweise $C_2$-$C_4$-Alkenyl, Cyanomethyl oder $C_1$-$C_4$-Alkoxy-$C_1$-$C_4$-Alkyl,

$R_4$ und $R_5$ sind Wasserstoff, $C_1$-$C_4$-Alkyl, $C_1$-$C_4$-Alkenyl, $C_1$-$C_4$-Alkoxy-$C_1$-$C_4$-alkyl, Phenyl oder $C_1$-$C_3$-Alkylphenyl, vorzugsweise Wasserstoff, Methyl oder Phenyl, wobei insbesondere $R_4$ Wasserstoff bedeutet, wenn $R_5$ kein Wasserstoff bedeutet, und

X ein Anion ist, beispielsweise Chlorid, Bromid, Iodid, Fluorid, Sulfat, Hydrogensulfat, Carbonat, Hydrogencarbonat, Phosphat, Mono- und Di-Hydrogenphosphat, Pyrophosphat, Metaphosphat, Nitrat, Methosulfat, Dodecylsulfat, Dodecylbenzolsulfonat, Phosphonat, Methylphosphonat, Methandisulfonat, Methylsulfonat, Ethansulfonat.

Besonders bevorzugt sind cyclische Ammonium-N-(cyanomethyl)-N-methylchloride und -sulfate mit 5 bis 6 Ringatomen, die zusätzlich Sauerstoff und/oder Stickstoff als Heteroatome enthalten, besonders Morpholinium-N-(cyanomethyl)-N-methylchlorid, Piperidinium-N-(cyanomethyl)-N-methylchlorid, N-Pyrrolidinium-N-(cyanomethyl)-N-methylchlorid, und Piperazinium-N,N'-(dicyanomethyl)-N,N'-dimethylchlorid, die alle sehr gute Peroxidaktivatoren sind, und hohe Stabilität aufweisen.

An Hand eines allgemeinen Beispiels sollen die Synthesewege für die kationischen Nitrile dieser Erfindung dargestellt werden.

1. Cyclisches sekundäres Amin, Natriumcyanid und Formaldehyd, vorzugsweise 36%ige Formalinlösung, werden in einem Ethanol/Wassergemisch zusammengegeben. Nach einer Reaktionszeit von 3 bis 7 Stunden bei Temperaturen zwischen 10 und 30°C, vorzugsweise bei 25°C wird dem Ansatz wäßrige Salzsäure zugesetzt. Die wäßrige Phase wird mit einem geeigneten organischen Lösungsmittel, z.B. Methylenchlorid extrahiert. Von den vereinten organischen Phasen wird das Lösungsmittel abgezogen. Das entstandene Aminoacetonitril wird in einem organischen Lösungsmittel aufgenommen und mit einem Methylierungsmittel wie Methylchlorid oder Dimethylsulfat bei Temperaturen zwischen 20 und 100°C, vorzugsweise zwischen 70 und 80°C zum entsprechenden Ammonium-N-(cyanomethyl)-N-methyl Salz umgesetzt.

2. Cyclisches sekundäres Amin und Natriumhydroxid wird in einem Toluol/Wasser Gemisch vorgelegt und bei Temperaturen zwischen 5 und 30°C, vorzugsweise bei 10 bis 15°C, Chloracetonitril zugetropft. Nach 2 bis 5 Stunden Reaktionszeit wird die organische Phase abgetrennt, die wäßrige Phase wird mit einem organischem Lösungsmittel extrahiert. Von den vereinten organischen Phasen wird das Lösungsmittel abgezogen. Das entstandene Aminoacetonitril wird in einem organischen Lösungsmittel aufgenommen und mit einem Methylierungsmittel wie Methylchlorid oder Dimethylsulfat bei Temperaturen zwischen 20 und 100°C, vorzugsweise zwischen 70 und 80°C zum entsprechenden Ammonium-N-(cyanomethyl)-N-methyl Salz umgesetzt.

3. Cyclisches tertiäres Amin und Chloracetonitril werden in einem geeigneten Lösungsmittel, z.B. in Aceton für 3 bis 7 Stunden bei Temperaturen zwischen 10 und 30°C, vorzugsweise bei 25°C zur Reaktion gebracht. Der entstandene Niederschlag, das entsprechende cyclische Ammonium-N-(cyanomethyl)-N-methylchlorid wird abfiltriert.

Gegenstand der Erfindung ist auch die Verwendung dieser Ammoniumnitrile als Bleichaktivatoren in bleichenden Wasch- und Reinigungsmitteln. Diese Wasch- und Reinigungsmittel enthalten neben einer Peroxidverbindung und den kationischen, nitrilischen Bleichaktivatoren üblicherweise auch oberflächenaktive Materialien und weitere bekannte Inhaltsstoffe.

Geeignete peroxidische Bleichmittel sind Alkaliperoxide, organische Peroxide wie Harnstoffperoxid, und anorganische Persalze, wie die Alkaliperborate, -percarbonate, -perphosphate, -persilicate und -persulfate. Mischungen aus zwei oder mehreren dieser Verbindungen sind ebenfalls geeignet. Besonders bevorzugt sind Natriumperborat-Tetrahydrat und insbesondere Natriumperborat-Monohydrat. Natriumperborat-Monohydrat ist wegen seiner guten Lagerbeständigkeit und seiner guten Löslichkeit in Wasser bevorzugt. Natriumpercarbonat kann aus Umweltschutzgründen bevorzugt sein.

Alkylhydroperoxide sind eine weitere geeignete Gruppe von Peroxidverbindungen. Beispiele für diese Stoffe sind Cumolhydroperoxid und t-Butylhydroperoxid.

In derartigen Wasch- und Reinigungsmitteln kann der erfindungsgemäße kationische, nitrilische Bleichaktivator mit einem Gewichtsanteil von etwa 0,1 % bis 20 %, bevorzugt von 0,5 % bis 10 %, insbesondere von 1 % bis 7,5 % vorhanden sein, zusammen mit einer Peroxidverbindung. Der Gewichtsanteil dieser Peroxidverbindungen beträgt gewöhnlich von 2 % bis 40 %, bevorzugt von 4 % bis 30 %, insbesondere von 10 % bis 25 %.

In den Wasch- und Reinigungsmitteln können neben den erfindungsgemäßen kationischen, nitrilischen Bleichaktivatoren noch andere geeignete Bleichaktivatoren, wie z.B. TAED enthalten sein.

Die oberflächenaktive Substanz kann von Naturprodukten abgeleitet sein, wie etwa Seife, oder ist eine synthetische Verbindung aus der Gruppe der anionischen, nichtionischen, amphoteren, zwitterionischen oder kationischen oberflächenaktiven Substanzen, oder Mischungen aus diesen. Viele geeignete Substanzen sind kommerziell erhältlich, und sind in der Literatur beschrieben, beispielsweise in "Surface active agents and detergents", Vol. 1 und 2, von Schwartz, Perry und Berch. Der Gesamtanteil der oberflächenaktiven Verbindungen kann bis zu 50 Gew.-% betragen,

vorzugsweise 1 Gew.-% bis 40 Gew.-%, insbesondere 4 Gew.-% bis 25 Gew.-%.

Synthetische anionische oberflächenaktive Substanzen sind üblicherweise wasserlösliche Alkalimetallsalze organischer Sulfate und Sulfonate mit Alkylresten von etwa 8 bis 22 Kohlenstoffatomen, wobei der Ausdruck "Alkyl" die Alkylsubstituenten höherer Arylreste einschließt.

Beispiele geeigneter anionischer Detergentien sind Natrium- und Ammoniumalkylsulfate, speziell die durch Sulfatierung höherer ($C_8$ bis $C_{18}$) Alkohole erhaltenen Sulfate; Natrium- und Ammoniumalkylbenzolsulfonate mit einem Alkylrest von $C_9$ bis $C_{20}$, insbesondere lineare sekundäre Natriumalkylbenzolsulfonate mit einem Alkylrest von $C_{10}$ bis $C_{15}$; Natriumalkylglycerinethersulfate, besonders die Ester der höheren, von Talg- und Kokosnußöl abgeleiteten Alkohole; die Natriumsulfate und -sulfonate der Kokosfettsäuremonoglyceride; Natrium- und Ammoniumsalze der Schwefelsäureester höherer ($C_9$ bis $C_{18}$) oxalkylierter, insbesondere der mit Ethylenoxid oxalkylierten Fettalkohole, die Reaktionsprodukte der Veresterung von Fettsäuren mit Isethionsäure und nachfolgender Neutralisierung mit Natriumhydroxid; Natrium- und Ammoniumsalze der Fettsäureamide des Methyltaurins; Alkan-Monosulfonate wie diejenigen aus der Reaktion von $\alpha$-Olefinen ($C_8$-$C_{20}$) mit Natriumbisulfit und diejenigen aus der Reaktion von Paraffinen mit $SO_2$ und $Cl_2$ mit anschließender basischer Hydrolyse, wobei ein Gemisch verschiedener Sulfonate entsteht; Natrium- und Ammoniumdialkylsulfosuccinate mit Alkylresten von $C_7$ bis $C_{12}$; und Olefinsulfonate, die bei der Reaktion von Olefinen, insbesondere $C_{10}$- bis $C_{20}$-$\alpha$-Olefinen, mit $SO_3$ und nachfolgender Hydrolyse der Reaktionsprodukte entstehen. Die bevorzugten anionischen Detergentien sind Natriumalkylbenzolsulfonate mit Alkylresten von $C_{15}$ bis $C_{18}$, und Natriumalkylethersulfate mit Alkylresten von $C_{16}$ bis $C_{18}$.

Beispiele für geeignete nichtionische oberflächenaktive Verbindungen, die bevorzugt zusammen mit anionischen oberflächenaktiven Verbindungen benutzt werden, sind insbesondere die Reaktionsprodukte von Alkylenoxiden (gewöhnlich Ethylenoxid), mit Alkylphenolen (Alkylreste von $C_5$ bis $C_{22}$), wobei die Reaktionsprodukte im allgemeinen 5 bis 25 Ethylenoxid(EO)-Einheiten im Molekül enthalten; die Reaktionsprodukte aliphatischer ($C_8$ bis $C_{18}$) primärer oder sekundärer, linearer oder verzweigter Alkohole mit Ethylenoxid, mit im allgemeinen 6 bis 30 EO; und die Additionsprodukte von Ethylenoxid an Reaktionsprodukte aus Propylenoxid und Ethylendiamin. Andere nichtionische oberflächenaktive Verbindungen sind Alkylpolyglycoside, langkettige tertiäre Aminoxide, langkettige tertiäre Phosphinoxide und Dialkylsulfoxide.

Amphotere oder zwitterionische oberflächenaktive Verbindungen können ebenfalls in den erfindungsgemäßen Zusammensetzungen verwendet werden, was aber wegen deren hoher Kosten meistens nicht erwünscht ist. Wenn amphotere oder zwitterionische Verbindungen verwendet werden, so geschieht das in der Regel in kleinen Mengen in Zusammensetzungen, die hauptsächlich anionische und nichtionische Tenside enthalten.

Auch Seifen können in den erfindungsgemäßen Zusammensetzungen verwendet werden, vorzugsweise mit einem Anteil von weniger als 25 Gew.-%. Sie sind besonders geeignet in geringen Mengen in binären (Seife/anionisches Tensid) oder in ternären Mischungen zusammen mit nichtionischen oder gemischten synthetischen anionischen und nichtionischen Tensiden. Die verwendeten Seifen sind bevorzugt die Natriumsalze, und weniger bevorzugt die Kaliumsalze gesättigter oder ungesättigter $C_{10}$- bis $C_{24}$-Fettsäuren, oder deren Mischungen. Die Anteile solcher Seifen können von 0,5 Gew.-% bis 25 Gew.-% betragen, geringere Mengen von 0,5 Gew.-% bis 5 Gew.-% sind im allgemeinen ausreichend zur Schaumkontrolle. Seifenanteile zwischen etwa 2 % und etwa 20 %, besonders zwischen etwa 5 % und etwa 10 %, haben einen positiven Effekt. Dieses ist besonders der Fall in hartem Wasser, wo die Seife als zusätzliche Buildersubstanz dient.

Die Wasch- und Reinigungsmittel enthalten im allgemeinen auch einen Builder. Als Builder kommen in Betracht: Calcium bindende Stoffe, Fällungsmittel, Calcium-spezifische Ionentauscher und deren Mischungen. Beispiele für Calcium bindende Stoffe umfassen Alkalimetallpolyphosphate, wie Natriumtripolyphosphat; Nitrilotriessigsäure und ihre wasserlöslichen Salze; die Alkalimetallsalze der Carboxymethyloxybernsteinsäure, Ethylendiamintetraessigsäure, Oxydibernsteinsäure, Mellithsäure, Benzolpolycarbonsäuren, Zitronensäure; und Polyacetalcarboxylate, wie in U.S. Pat. 4 144 226 und 4 146 495 offenbart.

Beispiele von Fällungsmitteln umfassen Natriumorthophosphat, Natriumcarbonat und Seifen aus langkettigen Fettsäuren.

Beispiele von Ionentauschern, die für Calcium spezifisch sind, umfassen die verschiedenen Arten wasserunlöslicher, kristalliner oder amorpher Aluminiumsilicate, von denen die Zeolithe die bekanntesten Vertreter sind.

Diese Buildersubstanzen können von 5 Gew.-% bis 80 Gew.-% vorhanden sein, bevorzugt ist ein Anteil von 10 Gew.-% bis 60 Gew.-%.

Neben den bereits erwähnten Inhaltsstoffen können die Wasch- und Reinigungsmittel jeden der konventionellen Zusatzstoffe in Mengen enthalten, die man üblicherweise in solchen Mitteln vorfindet. Beispiele dieser Zusatzstoffe umfassen Schaumbildner, wie etwa Alkanolamide, besonders die Monoethanolamide aus Palmkernöl-Fettsäuren und Kokosnuß-Fettsäuren; schaumverhindernde Substanzen, wie etwa Alkylphosphate und -silicone; Hilfsmittel,wie etwa Natriumcarboxymethylcellulose und Alkyl- oder substituierte Alkylcelluloseether; Stabilisatoren, wie Ethylendiamintetraessigsäure; Weichmacher für Textilien; anorganische Salze, wie Natriumsulfat; und, in üblicherweise kleinen Mengen, fluoreszierende Stoffe, Parfüme, Enzyme wie Proteasen, Cellulasen, Lipasen und Amylasen, Desinfektionsmittel und Farbstoffe. Die Bleichaktivatoren dieser Erfindung können in einer Vielzahl von Produkten eingesetzt werden.

Diese umfassen Textilwaschmittel, Textilbleichmittel, Oberflächenreiniger, Toilettenreiniger, Geschirrspülmaschinenreiniger, und auch Gebißreiniger. Die Waschmittel können in fester Form oder flüssiger Form vorliegen.

Es ist aus Gründen der Stabilität und Handhabbarkeit vorteilhaft, die Bleichaktivatoren in Form von Granulaten zu verwenden, die neben dem Bleichaktivator ein Bindemittel enthalten. Verschiedene Methoden, solche Granulate herzustellen, sind in der Patentliteratur beschrieben, so beispielsweise in Kanada Pat. Nr. 1 102 966, GB-1 561 333, US-4 087 369, EP-A-0 240 057, EP-A-0 241 962, EP-A-0 101 634 und EP-A-0 062 523. Jede dieser Methoden ist für die erfindungsgemäßen Bleichaktivatoren anwendbar.

Die die erfindungsgemäßen Bleichaktivatoren enthaltenden Granulate werden im allgemeinen der Waschmittelzusammensetzung zusammen mit den anderen, trockenen Bestandteilen wie etwa Enzymen, anorganischen Peroxidbleichmitteln zugesetzt. Die Waschmittelzusammensetzung, zu der die Aktivatorgranulate zugegeben werden, kann auf verschiedene Wegen erhalten werden, wie etwa Trockenmischen, Extrudieren, Sprühtrocknung.

In einer weiteren Ausführungsform sind die erfindungsgemäßen Bleichaktivatoren besonders geeignet für nicht wäßrige flüssige Waschmittel, zusammen mit einer bleichenden Peroxidverbindung, etwa Natriumperborat, um dem Waschmittel ein großes Reinigungsvermögen für Gewebe und Textilien zu verleihen. Derartige nicht wäßrige, flüssige Waschmittel, die pastöse und gelatinöse Detergentienzusammensetzungen mit einschließen, sind im Stand der Technik bekannt, und sind beispielsweise in US 2 864 770, US 2 940 938, US 4 772 412, US 3 368 977, GB-A-1205 711 GB-A-1 370 377, GB-A-1 270 040, GB-A-1 292 352, GB-A-2 194 536, DE-A-2 233 771, EP-A-0 028 849 beschrieben.

Es handelt sich dabei um Zusammensetzungen in Form eines nicht wäßrigen, flüssigen Mediums, in dem eine feste Phase dispergiert sein kann. Das nicht wäßrige, flüssige Medium kann eine flüssige, oberflächenaktive Substanz sein, vorzugsweise eine nichtionische oberflächenaktive Substanz; ein nicht polares flüssiges Medium wie etwa flüssiges Paraffin; ein polares Lösungsmittel, wie etwa Polyole, zum Beispiel Glycerin, Sorbitol, Ethylenglycol, eventuell in Verbindung mit niedermolekularen einwertigen Alkoholen wie Ethanol oder Isopropanol; oder Mischungen daraus.

Die feste Phase kann aus Buildersubstanzen, Alkalien, abrasiven Stoffen, Polymeren, Ionen, anderen festen ionischen oberflächenaktiven Stoffen, Bleichmitteln, fluoreszierenden Stoffen und anderen üblichen festen Inhaltsstoffen bestehen.

Die folgenden, nicht abschließenden Beispiele sollen einen Überblick über die Ausführungsformen der Erfindung geben.

Beispiel 1: Synthese von Piperidinoacetonitril

Zu 42,6 g Piperidin, vorgelegt bei unter 25°C, werden 40,6 g 36 % Formalinlösung unter Eiskühlung zugetropft. Das viskose Reaktionsgemisch wird mit 50 ml Wasser und 50 ml Ethanol verdünnt, danach werden 24,4 g Natriumcyanid zugesetzt. Das Reaktionsgemisch wird 2 bis 3 Stunden bei Raumtemperatur gerührt, dann werden 56,5 g 31 % Salzsäure unter Kühlung hinzugegeben. Die wäßrige Phase wird mit Methylenchlorid extrahiert, die organischen Phasen werden vereinigt, und das Lösungsmittel wird im Vakuum entfernt. Man erhält 59,4 g Piperidinoacetonitril, entsprechend 96 % Ausbeute.

Beispiel 2: Synthese von Piperidinoacetonitril

50 ml Wasser und 50 ml Toluol werden bei 10 bis 15°C vorgelegt. 21 g Natriumhydroxid werden unter Kühlung zugegeben. Nach Abkühlung auf 10 bis 15°C werden 43,4 g Piperidin zugesetzt, danach 37,8 g Chloracetonitril innerhalb 1,5 Stunden unter Kühlung zugetropft. Das viskose Reaktionsgemisch läßt man über Nacht stehen, und schüttelt dann die wäßrige Phase mit Toluol aus. Von den vereinigten organischen Phasen wird das Lösungsmittel abgezogen. Es entstehen 53,6 g Piperidinoacetonitril, entsprechend 86 % Ausbeute.

Beispiel 3: Synthese von Hexamethyleniminacetonitril

50 ml Wasser, 50 ml Methylenchlorid und 21 g Natriumhydroxid werden bei 15°C vorgelegt. Anschließend werden 49,6 g Hexamethylenimin bei 10 bis 15°C zugegeben, dann werden 37,8 g Chloracetonitril in 1,5 Stunden zugetropft. Der Ansatz wird 5 Stunden gerührt, die wäßrige Phase wird mit Methylenchlorid ausgeschüttelt, und aus den vereinigten organischen Phasen wird das Lösungsmittel entfernt. Man erhält 64,9 g Hexamethyleniminacetonitril, entsprechend 94 % Ausbeute.

Beispiel 4: Synthese von Piperidinium-N-(cyanomethyl)-N-methylchlorid

50 g Aceton und 38,8 g Chloracetonitril werden bei Raumtemperatur vorgelegt, 49,6 g 1-Methylpiperidin werden während 1 Stunde zugetropft. Da das Gemisch durch den entstehenden weißen Niederschlag sehr viskos wird, muß nach ca. 1,5 Stunden mit 100 ml Aceton verdünnt werden. Nach Beendigung der Reaktion wird das Produkt abgesaugt und mit Aceton gewaschen. Man erhält 80,3 g Piperidinium-N-(cyanomethyl)-N-methylchlorid, entsprechend 92 % Ausbeute.

Beispiel 5: Synthese von Pyrrolidinium-N-(cyanomethyl)-N-methylchlorid

50 ml Aceton und 38,8 g Chloracetonitril werden vorgelegt. Dazu tropft man unter Kühlung 43,9 g N-Methylpyrrolidin innerhalb 1,5 Stunden bei unter 25°C. Das Reaktionsgemisch wird trüb und viskos. Nach 5 Stunden Rühren wird das Produkt abgesaugt und mit Aceton gewaschen. Man erhält 76 g Pyrrolidinium-N-(cyanomethyl)-N-methylchlorid, entsprechend 95 % Ausbeute.

Beispiel 6: Synthese von Piperidinium-N-(cyanomethyl)-N-methylchlorid

124 g Piperidinoacetonitril werden in 160 g Isopropanol gelöst und in einem Autoklaven vorgelegt. Bei 70 bis 80°C wird Methylenchlorid mit einem Druck von 4 bis 5 bar aufgedrückt, die Reaktionszeit beträgt 24 Stunden. Gegen Ende der Reaktion erhält man eine viskose Lösung, aus der das Lösungsmittel im Vakuum entfernt wird. Das Produkt wird in quantitativer Ausbeute erhalten.

Beispiel 7:

Durch Zusammengeben von 200 ml einer wäßrigen Lösung von 5 g/l Referenzwaschmittel (WMP), erhalten von WFK-Testgewebe GmbH, Krefeld, 150 mg Natriumperborat-Monohydrat (PB*1) und 50 mg eines Aktivators wird eine Bleichmittelzusammensetzung erhalten. Vier mit schwarzem Tee verschmutzte Gewebestücke (BC-1-Tee auf Baumwolle, 1,25 g, WFK) werden für ein dreißigminütiges, isothermes Waschexperiment in einem Linitest-Gerät zugegeben. Die Gewebestücke werden nach der vorgegebenen Waschzeit mit Wasser gespült, getrocknet, gebügelt. Anschließend wird die Bleichwirkung mittels ELREPHO 2000 (Datacolor) festgestellt, indem die Unterschiede der Remissionen vor und nach dem Blechen ermittelt werden.

Das Experiment wurde mit verschiedenartigen Anschmutzungen (z.B. Gras, Curry) und bei verschiedenen Temperaturen (20°C, 40°C) wiederholt. Vergleichsversuche wurden durchgeführt, in denen 50 mg Tetraacetylethylendiamin (TAED) der Bleichmittelzusammensetzung zugegeben wurden. Ferner wurden die Experimente wiederholt unter Verwendung von jeweils 0,2 mmol Aktivator.

Es wurden die in Tabelle 1 angegebenen Bleichmittelzusammensetzungen mit den organischen Persäurevorstufen 1 bis 4 und dem Vergleichsaktivator 5 hergestellt. Ihre Wirksamkeit wurde durch den Vergleich der Remissionen des Gewebes vor und nach dem Bleichvorgang ermittelt. Die Resultate sind in Tabelle 1 angegeben. Die ΔΔR-Werte geben die Verbesserung der Bleichwirkung der erfindungsgemäßen Zusammensetzung verglichen mit PB*1 und TAED an:

$$\Delta\Delta R\ \text{QUAT-PB*1} = \Delta R\ (\text{QUAT}) - \Delta R\ (\text{PB*1})$$

$$\Delta\Delta R\ \text{QUAT-TAED} = \Delta R\ (\text{QUAT + TAED}) - \Delta R\ (\text{TAED})$$

Die Verbindungen 1 bis 5 sind

1 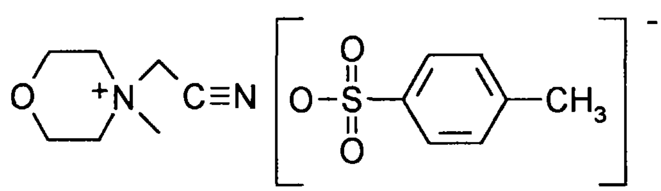

2 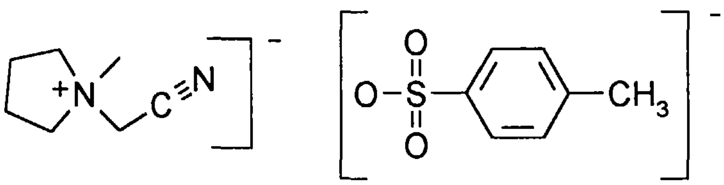

3 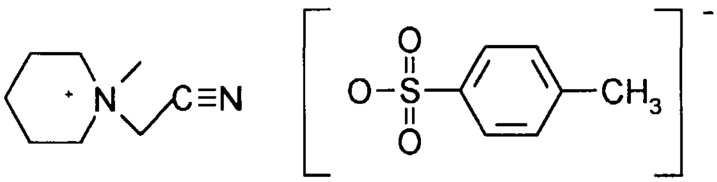

4 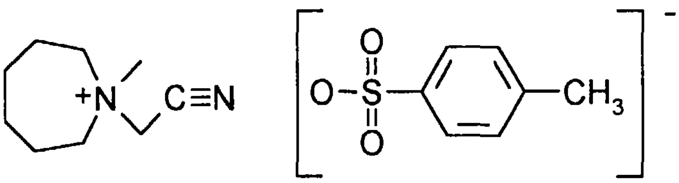

5 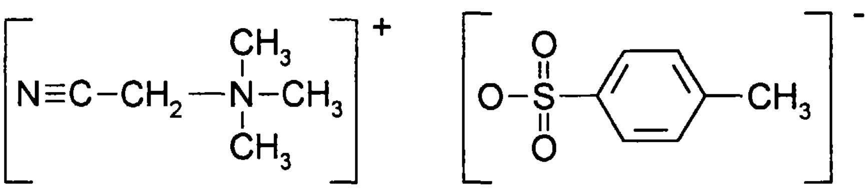

Tabelle 1

| Aktivator No. | ΔR (Quat) | ΔR (Quat + TAED) | ΔΔR (Quat - PB*1) | ΔΔR (Quat - TAED) | ΔΔR (Quat - PB*1) | ΔΔR (Quat - TAED) |
|---|---|---|---|---|---|---|
| PB*1 | 11,3 | | | | | |
| TAED | | 14,55 | | | | |
| 1 | 23,5 | 26,2 | 12,2 | 11,8 | 16,4 | 14,7 |
| 2 | 22,5 | 27,1 | 11,2 | 12,6 | 13,3 | 14,9 |
| 3 | 20,2 | 22,5 | 8,9 | 8,0 | 12,1 | 9,9 |
| 4 | 21,0 | 24,1 | 9,7 | 9,6 | 12,6 | 12,4 |
| 5 | 20,8 | 21,6 | 9,5 | 7,1 | 10,3 | 7,6 |

Die ersten 4 Spalten von Tabelle 1 beziehen sich auf einen Zusatz von 50 mg Aktivator, die letzten 2 Spalten auf die Zugabe von 0,2 mmol Aktivator.

Die stärkste Bleichwirkung tritt bei pH 9,5 bis 10 ein. Ein Vergleich der ΔR-Werte für die neuartigen kationischen Nitrile ergibt, daß die beste Schmutzentfernung bei einem beliebigen pH-Wert zwischen 7 und 12 eintritt.

Weitere nützliche Eigenschaften der kationischen Nitrile sind geringe Farbschädigung und geringe Faserschädigung. Experimente mit gefärbten Fasern (Remazol® rot gran. 133 und Remazol® schwarz B) zeigen, daß Aktivator 1

die geringsten Farbschädigungen hervorruft.

**Patentansprüche**

1. Verbindungen der allgemeinen Formel (I)

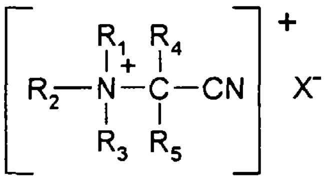

(I)

worin $R_1$ und $R_2$ zusammen mit dem Stickstoffatom, an das sie gebunden sind, einen Ring mit 4 bis 6 Kohlenstoffatomen bilden, der durch $C_1$- bis $C_5$-Alkyl, $C_1$- bis $C_5$-Alkoxy, $C_1$- bis $C_5$-Alkanoyl, Phenyl, Amino, Ammonium, Cyano, Cyanamino oder Chlor substituiert sein kann und wobei dieser Ring zusätzlich zum Stickstoffatom anstelle von Kohlenstoffatomen ein oder zwei Sauerstoff- oder Stickstoffatome, eine Gruppe N-$R_6$ oder eine Gruppe $R_3$-N-$R_6$ enthalten kann, worin $R_6$ Wasserstoff, $C_1$- bis $C_5$-Alkyl, $C_2$- bis $C_5$-Alkenyl, $C_2$- bis $C_5$-Alkinyl, Phenyl, $C_7$- bis $C_9$-Aralkyl, $C_5$- bis $C_7$-Cycloalkyl, $C_1$- bis $C_5$-Alkanoyl, Cyanomethyl oder Cyan ist,
$R_3$ ist $C_1$- bis $C_{24}$-, vorzugsweise $C_1$-$C_4$-Alkyl, $C_2$-$C_{24}$-, vorzugsweise $C_2$-$C_4$-Alkenyl, Cyanomethyl oder $C_1$-$C_4$-Alkoxy-$C_1$-$C_4$-Alkyl,
$R_4$ und $R_5$ sind Wasserstoff, $C_1$-$C_4$-Alkyl, $C_1$-$C_4$-Alkenyl, $C_1$-$C_4$-Alkoxy-$C_1$-$C_4$-alkyl, Phenyl oder $C_1$-$C_3$-Alkylphenyl, vorzugsweise Wasserstoff, Methyl oder Phenyl, wobei insbesondere Rest $R_4$ Wasserstoff bedeutet, wenn $R_5$ kein Wasserstoff bedeutet, und
X ein Anion ist.

2. Verbindungen der allgemeinen Formel I nach Anspruch 1, dadurch gekennzeichnet, daß $R_1$ und $R_2$ zusammen mit dem Stickstoffatom, an das sie gebunden sind, einen Ring mit 5 oder 6 Kohlenstoffatomen bilden, wobei ein Kohlenstoffatom durch ein Sauerstoffatom oder eine Gruppe N-$R_6$ ersetzt sein kann, wobei $R_6$ Cyanmethyl bedeutet, $R_3$ $C_1$-$C_4$-Alkyl, $R_4$ und $R_5$ Wasserstoff und X ein Anion bedeuten.

3. Verbindungen der allgemeinen Formel I nach Anspruch 1, dadurch gekennzeichnet, daß $R_1$ und $R_2$ zusammen mit dem Stickstoffatom, an das sie gebunden sind, einen Pyrrolidin-, Morpholin-, Piperidin- oder N'-Cyanomethyl-N'-methylpiperazinium-Ring bilden, $R_3$ $C_1$-$C_2$-Alkyl, $R_4$ und $R_5$ Wasserstoff und X ein Anion bedeuten.

4. Wasch- und Reinigungsmittel enthaltend eine Peroxidverbindung und eine Verbindung der Formel (I) gemäß Anspruch 1.

Europäisches Patentamt

# EUROPÄISCHER RECHERCHENBERICHT

Nummer der Anmeldung
EP 97 10 1830

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
| P,X | WO 96 40661 A (THE CLOROX COMPANY) 19.Dezember 1996<br>* Ansprüche 1-44 *<br>--- | 1-4 | C07D295/14<br>C07C255/03<br>C11D1/58<br>C11D1/62<br>C11D3/39 |
| X | J. ORG. CHEM.,<br>Bd. 22, Nr. 1, 5.März 1957,<br>Seiten 86-88, XP002014408<br>W. F. HART, M. E. MCGREAL: "Some new Quaternary-Substituted Alkyl Morpholinium Chlorides and Pyrrolidinium Alkyl Sulfates"<br>* Tabelle I *<br>--- | 1-3 | |
| X | KHIM.-FARM. ZH.,<br>Bd. 26, Nr. 7-8, 1992,<br>Seiten 60-62, XP000654261<br>GUBANOVA ET AL.: "Organic and organophosphorus derivatives of alpha-aminonitriles: synthesis and antiviral activity"<br>* Verbindung der Formel XIII *<br>--- | 1-3 | RECHERCHIERTE SACHGEBIETE (Int.Cl.6) |
| X | J. MED. CHEM.,<br>Bd. 17, Nr. 1, 1974,<br>Seiten 8-12, XP000654281<br>J. W. STANLEY ET AL.: "Synthesis and Enzymatic Evaluation of Some N-Alkyl Branched Chain Piperidine Salts and N-Alkyl-3-(N,N-diethylcarbamoyl)piperidine Salts as Inhibitors of Acetyl- and Butyrylcholinesterase"<br>* letzte Verbindung *<br>* Tabelle I *<br>---<br>-/-- | 1,2 | C07D<br>C07C<br>C11D |

Der vorliegende Recherchenbericht wurde für alle Patentansprüche erstellt

| Recherchenort | Abschlußdatum der Recherche | Prüfer |
|---|---|---|
| MÜNCHEN | 18.Juni 1997 | Herz, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 97 10 1830

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | BULL. SOC. CHIM. FR., Bd. 2, 1960, Seiten 383-389, XP000654234 A. LESPAGNOL ET AL.: "Guanidines monosubstituées à fonction ammonium quaternaire" * Seite 386 * | 1-3 | |
| | ----- | | RECHERCHIERTE SACHGEBIETE (Int.Cl.6) |

Der vorliegende Recherchenbericht wurde für alle Patentansprüche erstellt

| Recherchenort | Abschlußdatum der Recherche | Prüfer |
|---|---|---|
| MÜNCHEN | 18.Juni 1997 | Herz, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.........................................
& : Mitglied der gleichen Patentfamilie, übereinstimmendes Dokument

EPO FORM 1503 03.82 (P04C03)